# EUROPEAN PATENT APPLICATION

(11) **EP 4 302 798 A1**
(43) Date of publication of application: **10.01.2024**
(21) Application number: 23183950.7
(22) Date of filing: 06.07.2023
(51) Int. Cl.: A61M 5/14, A61M 5/145

(54) **MEDICAL INJECTOR**

(30) Priority: 08.07.2022 EP 22183893
(71) Applicant: Techmed Health Pte Ltd, Singapore 059817 (SG)
(72) Inventor: WIGHT, Brett, s259785 SINGAPORE (SG); SIMMONS, Adam, s259785 SINGAPORE (SG)
(74) Representative: Touroude & Associates

(57) **Abstract**

Medical injector configured to be coupled to a syringe containing a solution to be injected, wherein the injector is powered by a system presenting a potential mechanical energy, preferably a pre-stressed spring.

## Description

The present invention relates to the field of medical injectors.

Medical injectors can be used for injecting phase contrast before proceeding to an act of medical imaging. Medical injectors can also be used to inject a drug. Such devices allow for a precise control of the amount of injected product as well as of the injection speed and timing.

However, existing medical injectors need to be powered electrically which restrain their use dramatically. Indeed, medical imaging devices tend to become portable and are often moved directly to the patient's room. There is also an interest in using portable devices in places where there is no electricity, e.g. in developing countries or in war places. All these applications are not compatible with an electrically powered, cumbersome medical injector.

Furthermore, due to the metallic circuitry inherent to such devices, existing medical injectors cannot be placed inside an MRI device, which prevents easy real-time injection during the imaging process.

There is thus a need for a medical injector which is not electrically powered, which can be made portable and metal free.

The present invention answers this need and provides a medical injector configured to be coupled to a syringe containing a solution to be injected, wherein the injector is powered by a system presenting a potential mechanical energy, preferably a pre-stressed spring.

The injectors according to the invention can be used as backup injectors, or for testing purposes. The injectors according to the present invention can also be used as a mean to monitor precisely the fluid dynamics of drug delivery.

By "powered", it is meant that there is a mean which allows the injector to be put into motion and causes the plunger of the syringe to move so as to push the solution to be injected.

Said mean includes a system presenting potential mechanical energy. That is to say a system in which energy has been stored before use in a way which can allow the injector to be mechanically powered. Typically, said mean can comprise a pre-stressed spring. But it could also be a room filled with a pressured gas, two magnets maintained with similar poles faced together etc. Of course, any combination thereof can also be used.

Based on the amount of potential energy stored, it is possible to analogically program the injector so that it delivers an injection at a certain speed. It is also possible to provide a plurality of sources of potential energy so as to configure several injections with predetermined speed and pressure.

In a preferred embodiment, the injector is powered without any source of electricity. Indeed, sources of electricity usually include metallic parts which are unsuitable for MRI applications. Furthermore, electrical coupling always comes at the cost of efficiency and some energy is lost in the coupling. Potable electrical sources such as batteries plants the additional issue of sustainability as well as costs whereas wired sources of electricity hinder the portability of the device.

In a preferred embodiment of the present invention, the injector is metal free, preferably magnetically active metal free. The main technical advantage of a metal free injector is to allow compatibility with the strong magnetic fields of MRI. Typically austenitic stainless steel is compatible with MRI whereas ferritic and martensitic types of stainless are not due to their respective magnetic activities.

Another object of the present invention is a disposable syringe configured to be coupled with a medical injector according to the invention.

In order to provide a proper injection, it is important to provide the syringe with an air filtration system and a way to avoid the formation of bubbles which are often deleterious.

The present invention shall be better understood based on the following detailed description of a non-limiting embodiment of the present invention, and on the annexed drawing on which:
- figure 1 is a schematic view of a device according to the present invention,
- figure 2 is a lateral view of the device of figure 1, and
- figure 3 is a perspective view of the device according to figures 1 and 2.

For clarity sake, the relative sizes and proportions of the elements depicted on the drawing have not always been respected, it is understood that the depicted views are merely schematic.

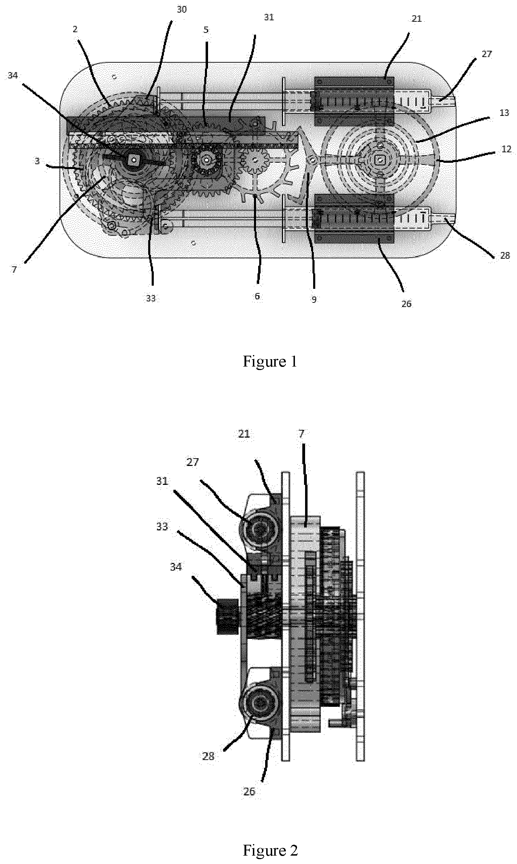

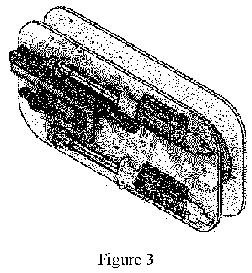

In an exemplary embodiment, the invention relates to an injector as depicted on figures 1, 2 and 3. The numerals have been omitted on purpose on figure 3 so as to keep the perspective clear.

The injector holds two syringes 27 and 28, configured to inject two different injection solutions. Both syringes are hold by a syringe holder 21, 26 into which they are clipped. The syringes 27, 28 are disposable and can be replaced upon use. During replacement, the syringe holders 21, 26 ensure the new syringes are correctly positioned.

Each distal end of the plungers of the syringes 27, 28, comes to a stop on a helical rack 30, 33 designed to push the plunger so as to cause the injection to proceed upon release of a pre-stressed main spring 7. The racks 30, 33 are placed on rack guides 31 which define the racks trajectories.

Said spring 7 is coupled to the racks through a ratchet 3, which is in turn coupled to an escape wheel 6 and a balance wheel 12 holding a helical balance spring 13.

The spring 7 is hold by a spring barrel 2 and can be pre-stressed upon winding up a winding key 34.

The balance wheel 12 and the balance spring 13 are meant to absorb part of the strength released by the main spring 7 so as to create a uniform injection speed. To do so, the balance spring 13 is connected to a fork 9 which is configured to brake the escape wheel 6.

The escape wheel is in turn connected to the main spring 7 through a gear train 5 and a network of spacers adequately positioned so as to prevent the different moving parts of the injector to hinder each other.

The winding key 34 can also be used to control the injection speed upon unwinding.

In this exemplary embodiment, all pieces are advantageously made of recyclable plastics, which allows MRI compatibility together with a low carbon footprint. In an alternative embodiments, the helical springs are made of a combination of non-ferromagnetic metals.

Although in the present embodiment both syringes are coupled to the same spring, in an alternative embodiment, each syringe may be coupled to a different system with a corresponding winding key.

Alternatively, the invention can comprise a clutch system so as to uncouple both syringes from a single spring.

The device according to the invention may comprise a pressure indicator so as to measure the stored energy. The indicator is advantageously analogical so as to avoid any battery of electricity sources and can for instance take the shape of a button

The device according to the exemplary embodiment is waterproof and made into materials which are inert to ethanol and to most disinfection solutions. Indeed, it is necessary for the device to withhold cleaning and disinfection protocols.

The invention is not limited to the described embodiments. Unless stated otherwise, the expression "comprising one" is to be understood as "comprising at least one" and "or" is to be understood as "and/or'.

## Claims

1. Medical injector configured to be coupled to a syringe containing a solution to be injected, wherein the injector is powered by a system presenting a potential mechanical energy, preferably a pre-stressed spring.

2. Medical injector according to claim 1, wherein the injector can be powered without any source of electricity.

3. Medical injector according to claim 1 or 2, wherein the injector is metal free.

4. Disposable syringe configured to be coupled with a medical injector according to any of the preceding claims.
